(19) Europäisches Patentamt / European Patent Office / Office européen des brevets

(11) **EP 3 409 192 A1**

(12) **EUROPEAN PATENT APPLICATION**
published in accordance with Art. 153(4) EPC

(43) Date of publication:
**05.12.2018 Bulletin 2018/49**

(51) Int Cl.:
*A61B 5/055* (2006.01)    *A61B 5/00* (2006.01)
*G01R 33/36* (2006.01)    *G01R 33/54* (2006.01)
*G01R 33/56* (2006.01)

(21) Application number: **16888286.8**

(22) Date of filing: **27.09.2016**

(86) International application number:
**PCT/KR2016/010811**

(87) International publication number:
**WO 2017/131310 (03.08.2017 Gazette 2017/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.01.2016 KR 20160011840**

(71) Applicants:
• **Samsung Electronics Co., Ltd.**
**Suwon-si, Gyeonggi-do 16677 (KR)**
• **Seoul National University R&DB Foundation Seoul 08826 (KR)**

(72) Inventors:
• **CHOI, Sang-cheon**
**Suwon-si**
**Gyeonggi-do 16484 (KR)**
• **ZHO, Sang-young**
**Suwon-si**
**Gyeonggi-do 16544 (KR)**
• **LEE, Jong-ho**
**Seoul 08826 (KR)**

(74) Representative: **D'Halleweyn, Nele Veerle Trees Gertrudis et al**
**Arnold & Siedsma**
**Bezuidenhoutseweg 57**
**2594 AC The Hague (NL)**

(54) **MAGNETIC RESONANCE IMAGING APPARATUS AND MAGNETIC RESONANCE IMAGE ACQUISITION METHOD THEREOF**

(57) Provided is a magnetic resonance imaging (MRI) apparatus including: a controller configured to determine a first slice and a second slice so that a difference between a slice number of the first slice of an object and a slice number of the second slice of the object is a predetermined interval value; and a radio frequency (RF) coil configured to transmit a preparation pulse signal including a frequency component enabling the first slice to be excited and transmit an RF signal including a plurality of frequency components so that both the first slice and the second slice are excited.

FIG. 3

100

**Description**

TECHNICAL FIELD

The present disclosure relates to a magnetic resonance imaging (MRI) apparatus and a magnetic resonance (MR) image acquisition method thereof, and more particularly, to an MRI apparatus for acquiring MR images by simultaneously acquiring MR signals of a plurality of slices.

BACKGROUND ART

Magnetic resonance imaging (MRI) refers to a process of producing an image by using information that is obtained through resonance after an atomic nucleus is exposed to a magnetic field. Resonance of an atomic nucleus refers to a phenomenon whereby, when a specific high-frequency wave enters an atomic nucleus magnetized by an external magnetic field, the atomic nucleus in a low energy state absorbs high-frequency energy and is excited to a high energy state. Atomic nuclei have different resonance frequencies depending on their types, and resonance is affected by the intensity of an external magnetic field. There are innumerous atomic nuclei in a human body, and hydrogen atomic nuclei are generally used for MRI.

When magnetic resonance (MR) images are acquired, there is a demand for technologies of processing MR images in a short time.

When information about a three-dimensional (3D) volume of an object is to be obtained in a short time, a method of acquiring a plurality of two-dimensional (2D) slice images in a direction of slices constituting the 3D volume is used. In this case, it is common to capture as many 2D slice images as the number of the slices.

In a multi-slice method, when each 2D slice image is acquired in a plurality of repetition time (TR) periods, an imaging time is reduced by alternately acquiring data about each 2D slice in the same TR period. That is, there exists a dead time when a TR period is much longer than an active time required for slice selection, phase encoding, and frequency encoding. In order to obtain information about another slice, the dead time may be used.

In parallel imaging, in order to reduce a scan time, a plurality of 2D slices are simultaneously excited to simultaneously acquire MR signals, through a plurality of coils, from the plurality of 2D slices, and the MR signals for the 2D slices are separated by using a difference in coil sensitivity information between the 2D slices. A 2D encoding method was used in conventional parallel imaging. The parallel imaging may correspond to a simultaneous multi-slice method. Representative examples of the parallel imaging include a sensitivity encoding (SENSE) method and a generalized autocalibrating partially parallel acquisition (GRAPPA) method.

DESCRIPTION OF EMBODIMENTS

SOLUTION TO PROBLEM

Provided are a magnetic resonance imaging (MRI) apparatus and a magnetic resonance (MR) image acquisition method thereof that acquire MR images having a plurality of contrasts by simultaneously exciting a plurality of slices.

An MRI apparatus according to an embodiment includes: a controller configured to determine a first slice and a second slice so that a difference between a slice number of the first slice of an object and a slice number of the second slice is a predetermined interval value; and a radio frequency (RF) coil configured to transmit a preparation pulse signal including a frequency component enabling the first slice to be excited and transmit an RF signal including a plurality of frequency components so that both the first slice and the second slice are excited.

BRIEF DESCRIPTION OF DRAWINGS

FIG. 1 is a block diagram of a general magnetic resonance imaging (MRI) system.
FIG. 2 is a block diagram of a communication unit according to an embodiment.
FIG. 3 is a schematic diagram of an MRI apparatus according to an embodiment.
FIG. 4 is a diagram of an MRI apparatus according to an embodiment.
FIG. 5 is a schematic flowchart of an MRI method according to an embodiment.
FIG. 6 is a flowchart of an MRI method according to an embodiment.
FIG. 7 is a pulse sequence schematic diagram according to an embodiment.
FIG. 8 is a diagram illustrating a plurality of slices of an object according to an embodiment.
FIG. 9 illustrates tables each showing an order of selecting a first slice and a second slice according to an embodiment.

BEST MODE

A magnetic resonance imaging (MRI) apparatus according to an embodiment includes: a controller configured to determine a first slice and a second slice so that a difference between a slice number of the first slice of an object and a slice number of the second slice of the object is a predetermined interval value; and a radio frequency (RF) coil configured to transmit a preparation pulse signal including a frequency component enabling the first slice to be excited and transmit an RF signal including a plurality of frequency components so that both the first slice and the second slice are excited.

Also, the controller may be further configured to determine the first slice and the second slice so that when the first slice is an even-numbered slice, the second slice is an odd-numbered slice, and when the first slice is an odd-numbered slice, the second slice is an even-numbered slice.

Also, the controller may be further configured to determine the first slice and the second slice so that the difference between the slice number of the first slice and the slice number of the second slice is half a total number of slices constituting the object.

Also, the RF coil may be further configured to receive magnetic resonance (MR) signals of the first slice and the second slice, wherein the MRI apparatus further includes an image processor configured to acquire an MR image having a first contrast for the first slice and an MR image having a second contrast for the second slice.

Also, the RF coil may be further configured to transmit an inverted RF pulse signal including a frequency component enabling the first slice to be excited.

Also, the image processor may be further configured to acquire a fluid-attenuated inversion recovery (FLAIR) image for the first slice and acquire at least one image from among a T1-weighted image and a T2-weighted image for the second slice.

Also, the image processor may be further configured to sequentially acquire images having the first contrast for a plurality of slices including the first slice, and simultaneously acquire images having the second contrast for slices respectively corresponding to the plurality of slices in a state where the images having the second contrast overlap the images having the first contrast, wherein a difference between a slice number of each of the corresponding slices and a slice number of each of the plurality of slices is the predetermined interval value.

Also, each of the images having the first contrast may be a FLAIR image, and each of the images having the second contrast may be a T1-weighted image or a T2-weighted image.

Also, the predetermined interval value may be a value obtained by dividing a total number of slices constituting the object by a multi-band acceleration factor.

A magnetic resonance imaging (MRI) method according to an embodiment includes: determining a first slice and a second slice of an object; transmitting a preparation pulse signal including a frequency component enabling the first slice to be excited; and transmitting a radio frequency (RF) signal including a plurality of frequency components so that both the first slice and the second slice are excited, wherein the determining includes determining the first slice and the second slice so that a difference between a slice number of the first slice and a slice number of the second slice is a predetermined interval value.

Also, the determining may include determining the first slice and the second slice so that when the first slice is an even-numbered slice, the second slice is an odd-numbered slice, and when the first slice is an odd-numbered slice, the second slice is an even-numbered slice.

Also, the determining may include determining the first slice and the second slice so that the difference between the slice number of the first slice and the slice number of the second slice is half a total number of slices constituting the object.

Also, the MRI method of claim 10 may further include: receiving magnetic resonance (MR) signals of the first slice and the second slice; and acquiring an MR image having a first contrast for the first slice and an MR image having a second contrast for the second slice.

Also, the transmitting of the preparation pulse signal may include transmitting an inverted RF pulse signal including a frequency component enabling the first slice to be excited.

Also, the acquiring may include: acquiring a fluid-attenuated inversion recovery (FLAIR) image for the first slice; and acquiring at least one image from among a T1-weighted image and a T2-weighted image for the second slice.

Also, the MRI method may further include sequentially acquiring images having the first contrast for a plurality of slices including the first slice, wherein the sequentially acquiring includes simultaneously acquiring images having the second contrast for slices respectively corresponding to the plurality of slices in a state where the images having the second contrast overlap the images having the first contrast, wherein a difference between a slice number of each of the corresponding slices and a slice number of each of the plurality of slices is the predetermined interval value. Also, each of the images having the first contrast may be a FLAIR image, and each of the images having the second contrast may be a T1-weighted image or a T2-weighted image.

Also, the predetermined interval value may be a value obtained by dividing a total number of slices constituting the object by a multi-band acceleration factor.

There is provided a computer-readable recording medium having embodied thereon a program for executing the MRI method according to an embodiment.

MODE OF DISCLOSURE

Advantages and features of the present disclosure and methods of accomplishing the same may be understood more readily by reference to the following detailed description of embodiments and the accompanying drawings. However, the present embodiments may have different forms and should not be construed as being limited to the descriptions set forth herein. Rather, these embodiments are provided so that this disclosure will be thorough and complete and will fully convey the scope of the present embodiments to one of ordinary skill in the art, and the present disclosure will only be defined by the appended claims.

Terms used herein will now be briefly described and then one or more embodiments will be described in detail.

The terms used in the present disclosure are selected from among common terms that are currently widely used in consideration of their functions in the present disclosure. However, the terms may vary according to the intention of one of ordinary skill in the art, a precedent, or the advent of new technology. Also, in particular cases, some terms are discretionally selected by the applicant, and in this case, the meaning of the selected terms will be described in detail in the detailed description of the disclosure. Thus, the terms used herein have to be defined based on the meaning of the terms together with the description throughout the specification.

When a part "includes" or "comprises" an element, unless there is a particular description contrary thereto, the part may further include other elements, not excluding the other elements. Also, the term "unit" in the embodiments of the present disclosure means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may include any one or more of components such as software components, object-oriented software components, class components, and task components, processes, functions, attributes, procedures, subroutines, segments of program code, drivers, firmware, micro codes, circuits, data, a database, data structures, tables, arrays, and variables. Functions provided by the components and "units" may be combined into a smaller number of components and "units", or may be divided into additional components and "units".

Hereinafter, embodiments of the present disclosure will be described in detail with reference to the accompanying drawings in order to enable one of ordinary skill in the art to easily embody and practice the present disclosure. Also, parts in the drawings unrelated to the detailed description are omitted to ensure clarity of the present disclosure.

In the present specification, an "image" may refer to multi-dimensional data composed of discrete image elements (e.g., pixels in a two-dimensional (2D) image and voxels in a three-dimensional (3D) image). For example, the image may include a medical image of an object captured by an X-ray apparatus, a computed tomography (CT) apparatus, a magnetic resonance imaging (MRI) apparatus, an ultrasound diagnosis apparatus, or another medical imaging apparatus.

Furthermore, in the present specification, an "object" may be a human, an animal, or a part of a human or an animal. For example, the object may include an organ (e.g., the liver, heart, womb, brain, breast, or abdomen), or a blood vessel. Furthermore, the "object" may be a phantom. The phantom means a material having a density, an effective atomic number, and a volume that are approximately the same as those of an organism. For example, the phantom may be a spherical phantom having properties similar to a physical body.

Furthermore, in the present specification, a "user" may be, but is not limited to, a medical expert such as a medical doctor, a nurse, a medical laboratory technologist, a medical imaging specialist, or a technician who repairs a medical apparatus.

Furthermore, in the present specification, an "MR image" refers to an image of an object acquired by using the nuclear magnetic resonance principle.

Furthermore, in the present specification, a "pulse sequence" refers to continuity of signals repeatedly applied by an MRI apparatus. The pulse sequence may include a time parameter of a radio frequency (RF) pulse, for example, repetition time (TR) or echo time (TE).

Furthermore, in the present specification, a "pulse sequence schematic diagram" shows an order of events that occur in an MRI apparatus. For example, the pulse sequence schematic diagram may be a diagram showing an RF pulse, a gradient magnetic field, an MR signal, or the like according to time.

Also, a "multi-band acceleration factor" may refer to the number of images to be simultaneously acquired in a single sequence. For example, when the multi-band acceleration factor is 2, a pulse sequence may include two frequency components. Accordingly, when the multi-band acceleration factor is 2, two MR images may be simultaneously acquired in an overlapping state.

An MRI system is an apparatus for acquiring a sectional image of a part of an object by expressing, in a contrast comparison, a strength of an MR signal with respect to an RF signal generated in a magnetic field having a specific strength. For example, if an RF signal that only resonates a specific atomic nucleus (for example, a hydrogen atomic nucleus) is emitted for an instant toward the object placed in a strong magnetic field and then such emission stops, an MR signal is emitted from the specific atomic nucleus, and thus the MRI system may receive the MR signal and acquire an MR image. The MR signal denotes an RF signal emitted from the object. An intensity of the MR signal may be determined according to a density of a predetermined atom (for example, hydrogen) of the object, a relaxation time T1, a relaxation time T2, and a flow of blood or the like. MRI systems include characteristics different from those of other imaging apparatuses. Unlike imaging apparatuses such as CT apparatuses that acquire images according to a direction of detection hardware, MRI systems may acquire 2D images or 3D volume images that are oriented toward an optional point. MRI systems do not expose objects or examiners to radiation, unlike CT apparatuses, X-ray apparatuses, position emission tomography (PET) apparatuses, and single photon emission CT (SPECT) apparatuses, may acquire images having a high soft tissue contrast, and may acquire neurological images, intravascular images, musculoskeletal images, and oncologic images that are required to precisely capture abnormal tissues.

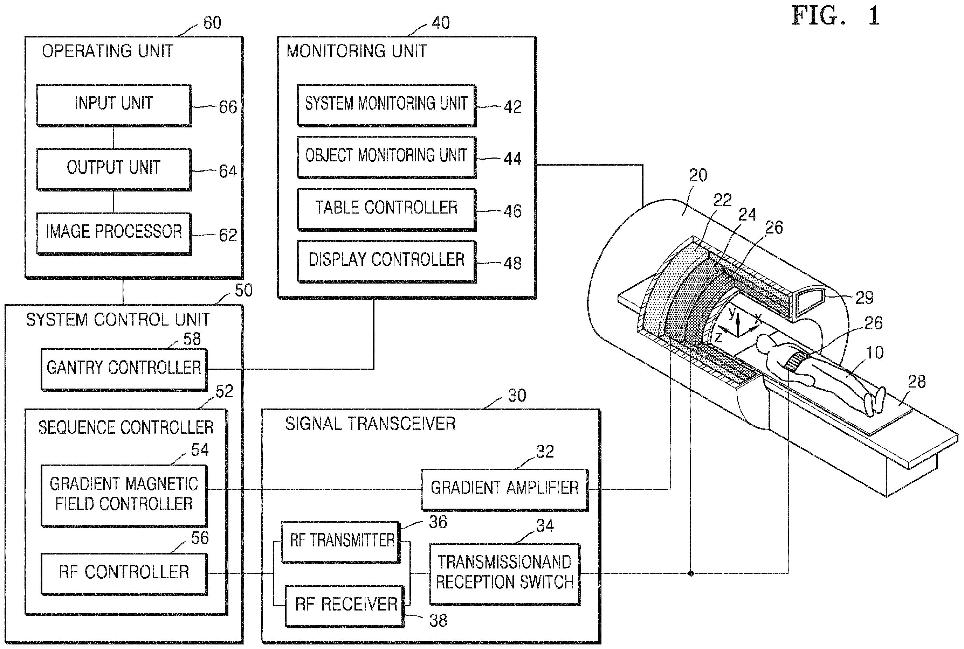

FIG. 1 is a block diagram of a general MRI system. Referring to FIG. 1, the general MRI system may include a gantry 20, a signal transceiver 30, a monitoring unit 40, a system control unit 50, and an operating unit 60.

The gantry 20 prevents external emission of electromagnetic waves generated by a main magnet 22, a gradient coil 24, and an RF coil 26. A magnetostatic field and a gradient magnetic field are formed in a bore in the gantry 20, and an RF signal is emitted toward an object 10.

The main magnet 22, the gradient coil 24, and the RF coil 26 may be arranged in a predetermined direction of the gantry 20. The predetermined direction may be a coaxial cylinder direction. The object 10 may be disposed on a table 28 that is capable of being inserted into a cylinder along a horizontal axis of the cylinder.

The main magnet 22 generates a magnetostatic field or a static magnetic field for aligning magnetic dipole moments of atomic nuclei of the object 10 in a constant direction. A more precise and accurate MR image of the object 10 may be acquired as a magnetic field generated by the main magnet 22 is stronger and more uniform.

The gradient coil 24 includes X, Y, and Z coils for generating gradient magnetic fields in X-, Y-, and Z-axis directions crossing each other at right angles. The gradient coil 24 may provide location information of each region of the object 10 by differently inducing resonance frequencies according to the regions of the object 10.

The RF coil 26 may emit an RF signal toward a patient and receive an MR signal emitted from the patient. In detail, the RF coil 26 may transmit, toward atomic nuclei included in the patient and having precessional motion, an RF signal having the same frequency as that of the precessional motion, stop transmitting the RF signal, and then receive an MR signal emitted from the atomic nuclei included in the patient.

For example, in order to transit an atomic nucleus from a low energy state to a high energy state, the RF coil 26 may generate and apply an electromagnetic wave signal having an RF corresponding to a type of the atomic nucleus, for example, an RF signal, to the object 10. When the electromagnetic wave signal generated by the RF coil 26 is applied to the atomic nucleus, the atomic nucleus may transit from the low energy state to the high energy state. Then, when electromagnetic waves generated by the RF coil 26 disappear, the atomic nucleus to which the electromagnetic waves were applied transits from the high energy state to the low energy state, thereby emitting electromagnetic waves having a Lamor frequency. In other words, when the applying of the electromagnetic wave signal to the atomic nucleus is stopped, an energy level of the atomic nucleus is changed from a high energy level to a low energy level, and thus the atomic nucleus may emit electromagnetic waves having a Lamor frequency. The RF coil 26 may receive electromagnetic wave signals from atomic nuclei included in the object 10.

The RF coil 26 may be realized as one RF transmitting and receiving coil having both a function of generating electromagnetic waves having an RF corresponding to a type of an atomic nucleus and a function of receiving electromagnetic waves emitted from an atomic nucleus. Alternatively, the RF coil 26 may be realized as a transmission RF coil having a function of generating electromagnetic waves having an RF corresponding to a type of an atomic nucleus, and a reception RF coil having a function of receiving electromagnetic waves emitted from an atomic nucleus. Also, the RF coil 26 may be fixed to the gantry 20 or may be detachable. When the RF coil 26 is detachable, the RF coil 26 may be an RF coil for a part of the object 10, such as a head RF coil, a chest RF coil, a leg RF coil, a neck RF coil, a shoulder RF coil, a wrist RF coil, or an ankle RF coil.

The RF coil 26 may communicate with an external apparatus via wires and/or wirelessly, and may also perform dual tune communication according to a communication frequency band.

Also, the RF coil 26 may be a birdcage coil, a surface coil, or a transverse electromagnetic (TEM) coil according to coil structures.

Also, the RF coil 26 may be a transmission exclusive coil, a reception exclusive coil, or a transmission and reception coil according to methods of transmitting and receiving an RF signal. Also, the RF coil 26 may be an RF coil having various numbers of channels, such as 16 channels, 32 channels, 72 channels, or 144 channels.

The gantry 20 may further include a display 29 disposed outside the gantry 20 and a display (not shown) disposed inside the gantry 20. The gantry 20 may provide predetermined information to a user or the object 10 through the display 29 and the display respectively disposed outside and inside the gantry 20.

The signal transceiver 30 may control the gradient magnetic field formed inside the gantry 20, i.e., in the bore, according to a predetermined MR sequence, and control transmission and reception of an RF signal and an MR signal.

The signal transceiver 30 may include a gradient amplifier 32, a transmission and reception switch 34, an RF transmitter 36, and an RF receiver 38.

The gradient amplifier 32 drives the gradient coil 24 included in the gantry 20, and may supply a pulse signal for generating a gradient magnetic field to the gradient coil 24 under the control of a gradient magnetic field controller 54. By controlling the pulse signal supplied from the gradient amplifier 32 to the gradient coil 24, gradient magnetic fields in X-, Y-, and Z-axis directions may be synthesized.

The RF transmitter 36 and the RF receiver 38 may drive the RF coil 26. The RF transmitter 36 may supply an RF pulse in a Lamor frequency to the RF coil 26, and the RF receiver 38 may receive an MR signal received by the RF coil 26.

The transmission and reception switch 34 may adjust transmitting and receiving directions of the RF signal and the MR signal. For example, the transmission and reception switch 34 may emit the RF signal toward the object 10 through the RF coil 26 during a transmission mode, and receive the MR signal from the object 10 through the RF coil 26 during a reception mode. The transmission and reception switch 34 may be controlled by a control signal output by an RF controller 56.

The monitoring unit 40 may monitor or control the gantry 20 or devices mounted on the gantry 20. The monitoring unit 40 may include a system monitoring unit 42, an object monitoring unit 44, a table controller 46, and a display controller 48.

The system monitoring unit 42 may monitor and control a state of the magnetostatic field, a state of the gradient magnetic field, a state of the RF signal, a state of the RF coil 26, a state of the table 28, a state of a device for measuring body information of the object 10, a power supply state, a state of a thermal exchanger, and a state of a compressor.

The object monitoring unit 44 monitors a state of the object 10. In detail, the object monitoring unit 44 may include a camera for observing a movement or position of the object 10, a respiration measurer for measuring the respiration of the object 10, an electrocardiogram (ECG) measurer for measuring the electrical activity of the object 10, or a temperature measurer for measuring a temperature of the object 10.

The table controller 46 controls a movement of the table 28 where the object 10 is positioned. The table controller 46 may control the movement of the table 28 according to a sequence control of a sequence controller 52 comprised in the sequence control unit 50. For example, during moving imaging of the object 10, the table controller 46 may continuously or discontinuously move the table 28 according to the sequence control of the system control unit 50, and thus the object 10 may be imaged in a field of view (FOV) larger than that of the gantry 20.

The display controller 48 controls the display 29 disposed outside the gantry 20 and the display disposed inside the gantry 20. In detail, the display controller 48 may control the display 29 and the display to be on or off, and may control a screen image to be output on the display 29 and the display. Also, when a speaker is located inside or outside the gantry 20, the display controller 48 may control the speaker to be on or off, or may control sound to be output via the speaker.

The system control unit 50 may include a sequence controller 52 for controlling a sequence of signals formed in the gantry 20, and a gantry controller 58 for controlling the gantry 20 and the devices mounted on the gantry 20.

The sequence controller 52 may include the gradient magnetic field controller 54 for controlling the gradient amplifier 32, and the RF controller 56 for controlling the RF transmitter 36, the RF receiver 38, and the transmission and reception switch 34. The sequence controller 52 may control the gradient amplifier 32, the RF transmitter 36, the RF receiver 38, and the transmission and reception switch 34 according to a pulse sequence received from the operating unit 60. Here, the pulse sequence includes all information required to control the gradient amplifier 32, the RF transmitter 36, the RF receiver 38, and the transmission and reception switch 34. For example, the pulse sequence may include information about a strength, an application time, and application timing of a pulse signal applied to the gradient coil 24.

The operating unit 60 may request the system control unit 50 to transmit pulse sequence information while controlling an overall operation of the MRI system.

The operating unit 60 may include an image processor 62 for receiving and processing the MR signal received by the RF receiver 38, an output unit 64, and an input unit 66.

The image processor 62 may process the MR signal received from the RF receiver 38 so as to generate MR image data of the object 10.

The image processor 62 receives the MR signal received by the RF receiver 38 and performs any one of various signal processes, such as amplification, frequency transformation, phase detection, low frequency amplification, and filtering, on the received MR signal.

The image processor 62 may arrange digital data in a k-space (for example, also referred to as a Fourier space or a frequency space) of a memory, and rearrange the digital data into image data via 2D or 3D Fourier transformation.

Also, the image processor 62 may perform a composition process or a difference calculation process on the image data if required. The composition process may be an addition process performed on a pixel or a maximum intensity projection (MIP) process. The image processor 62 may store not only the rearranged image data but also image data on which a composition process or a difference calculation process is performed, in a memory (not shown) or an external server. The image processor 62 may perform any of the signal processes on the MR signal in parallel. For example, the image processor 62 may perform a signal process on a plurality of MR signals received by a multi-channel RF coil in parallel so as to rearrange the plurality of MR signals into image data.

The output unit 64 may output image data generated or rearranged by the image processor 62 to the user. The output unit 64 may also output information required for the user to manipulate the MRI system, such as a user interface (UI), user information, or object information. The output unit 64 may be a speaker, a printer, a cathode-ray tube (CRT) display, a liquid crystal display (LCD), a plasma display panel (PDP) display, an organic light-emitting device (OLED) display, a field emission display (FED), a light-emitting diode (LED) display, a vacuum fluorescent display (VFD), a digital light processing (DLP) display, a flat panel display (FPD), a 3D display, a transparent display, or any one of other various output devices that are well known to one of ordinary skill in the art.

The user may input object information, parameter information, a scan condition, a pulse sequence, or information about image composition or difference calculation by using the input unit 66. The input unit 66 may be a keyboard, a mouse, a track ball, a voice recognizer, a gesture recognizer, a touch screen, or any one of other various input devices that are well known to one of ordinary skill in the art.

The signal transceiver 30, the monitoring unit 40, the system control unit 50, and the operating unit 60 are separate components in FIG. 1, but it will be obvious to one of ordinary skill in the art that respective functions of the signal transceiver 30, the monitoring unit 40, the system control unit 50, and the operating unit 60 may be performed by another component. For example, the image processor 62 converts the MR signal received from the RF receiver 38 into a digital signal in FIG. 1, but alternatively, the conversion of the MR signal into the digital signal may be directly performed by the RF receiver 38 or the RF coil 26.

The gantry 20, the RF coil 26, the signal transceiver 30, the monitoring unit 40, the system control unit 50, and the operating unit 60 may be connected to each other by wire or wirelessly, and when they are connected wirelessly, the MRI system may further include an apparatus (not shown) for synchronizing clock signals therebetween. Communication between the gantry 20, the RF coil 26, the signal transceiver 30, the monitoring unit 40, the system control unit 50, and the operating unit 60 may be performed by using a high-speed digital interface such as low voltage differential signaling (LVDS), asynchronous serial communication such as a universal asynchronous receiver transmitter (UART), a low-delay network protocol such as error synchronous serial communication or a controller area network (CAN), optical communication, or any of other various communication methods that are well known to one of ordinary skill in the art.

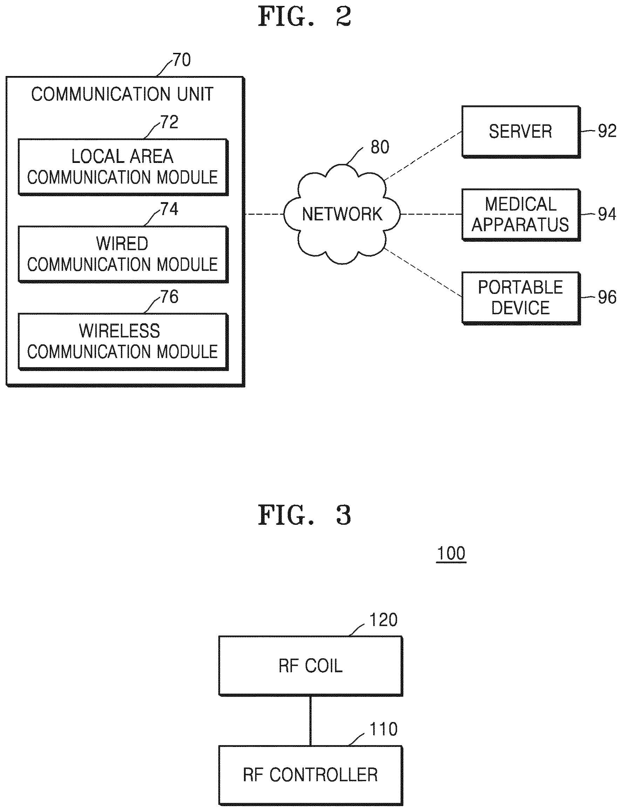

FIG. 2 is a block diagram of a communication unit 70 according to an embodiment. Referring to FIG. 2, the communication unit 70 may be connected to at least one selected from the gantry 20, the signal transceiver 30, the monitoring unit 40, the system control unit 50, and the operating unit 60 of FIG. 1.

The communication unit 70 may transmit and receive data to and from a hospital server or another medical apparatus in a hospital, which is connected through a picture archiving and communication system (PACS), and perform data communication according to the digital imaging and communications in medicine (DICOM) standard.

As shown in FIG. 2, the communication unit 70 may be connected to a network 80 by wire or wirelessly to communicate with a server 92, a medical apparatus 94, or a portable device 96.

In detail, the communication unit 70 may transmit and receive data related to the diagnosis of an object through the network 80, and may also transmit and receive a medical image captured by the medical apparatus 94, such as a CT apparatus, an MRI apparatus, or an X-ray apparatus. In addition, the communication unit 70 may receive a diagnosis history or a treatment schedule of the object from the server 92 and use the same to diagnose the object. The communication unit 70 may perform data communication not only with the server 92 or the medical apparatus 94 in a hospital, but also with the portable device 96, such as a mobile phone, a personal digital assistant (PDA), or a laptop of a doctor or patient.

Also, the communication unit 70 may transmit information about a malfunction of the MRI system or about medical image quality to a user through the network 80, and receive a feedback regarding the information from the user.

The communication unit 70 may include at least one component enabling communication with an external apparatus. For example, the communication unit 70 may include a local area communication module 72, a wired communication module 74, and a wireless communication module 76.

The local area communication module 72 refers to a module for performing local area communication with an apparatus within a predetermined distance. Examples of local area communication technology according to an embodiment include, but are not limited to, a wireless local area network (LAN), Wi-Fi, Bluetooth, ZigBee, Wi-Fi direct (WFD), ultra wideband (UWB), infrared data association (IrDA), Bluetooth low energy (BLE), and near field communication (NFC).

The wired communication module 74 refers to a module for performing communication by using an electric signal or an optical signal. Examples of wired communication technology according to an embodiment include wired communication techniques using a twisted pair cable, a coaxial cable, and an optical fiber cable, and other well known wired communication techniques.

The wireless communication module 76 transmits and receives a wireless signal to and from at least one selected from a base station, an external apparatus, and a server in a mobile communication network. Here, the wireless signal may be a voice call signal, a video call signal, or data in any one of various formats according to transmission and reception of a text/multimedia message.

FIG. 3 is a schematic diagram of an MRI apparatus 100 according to an embodiment. The MRI apparatus 100 includes a controller 110 and an RF coil 120.

The controller 110 of FIG. 3 may correspond to the RF controller 56 of FIG. 1, and the RF coil 120 of FIG. 3 may correspond to the RF coil 26 of FIG. 1.

The controller 110 determines a first slice and a second slice so that a difference between a slice number of the first slice of an object and a slice number of the second slice of the object is a predetermined interval value.

In an embodiment, the first slice and the second slice are included in a plurality of slices of the object. The plurality of slices of the object may refer to two or more slices from among 2D slices constituting a volume of the object.

The predetermined interval value may be a value set to maintain constant an interval between the first slice and the second slice. In detail, the predetermined interval value may be a value set to maintain the interval between the first slice and the second slice at a predetermined distance or more.

For example, the predetermined interval value may be a value obtained by dividing a total number of slices constituting the object by a multi-band acceleration factor. For example, when the multi-band acceleration factor is 2, the predetermined interval value may be half the total number of the slices constituting the object.

The controller 110 may reduce the influence of a preparation pulse for the first slice on the second slice by maintaining the interval between the first slice and the second slice at the predetermined distance or more.

In an embodiment, when the first slice is an even-numbered slice, the second slice may be an odd-numbered slice, and when the first slice is an odd-numbered slice, the second slice may be an even-numbered slice.

The RF coil 120 transmits a preparation pulse signal including a frequency component that enables the first slice to be excited.

Also, the RF coil 120 transmits an RF signal including a plurality of frequency components so that both the first slice and the second slice are excited.

Excitation may refer to the transition to a high energy state of magnetized atomic nuclei by absorbing high-frequency energy when an RF signal is transmitted to the magnetized nuclei. Excitation of a slice may refer to the transition from a low energy state to a high energy state of atomic nuclei included in the slice when an electromagnetic wave signal is applied to the atomic nuclei.

Excitation of a plurality of slices together may include simultaneous excitation of the plurality of slices.

The plurality of frequency components may respectively correspond to the plurality of slices. In detail, when the RF signal includes an A frequency component and a B frequency component, the A frequency component may excite the first slice from among the plurality of slices, and the B frequency component may excite the second slice from among the plurality of slices.

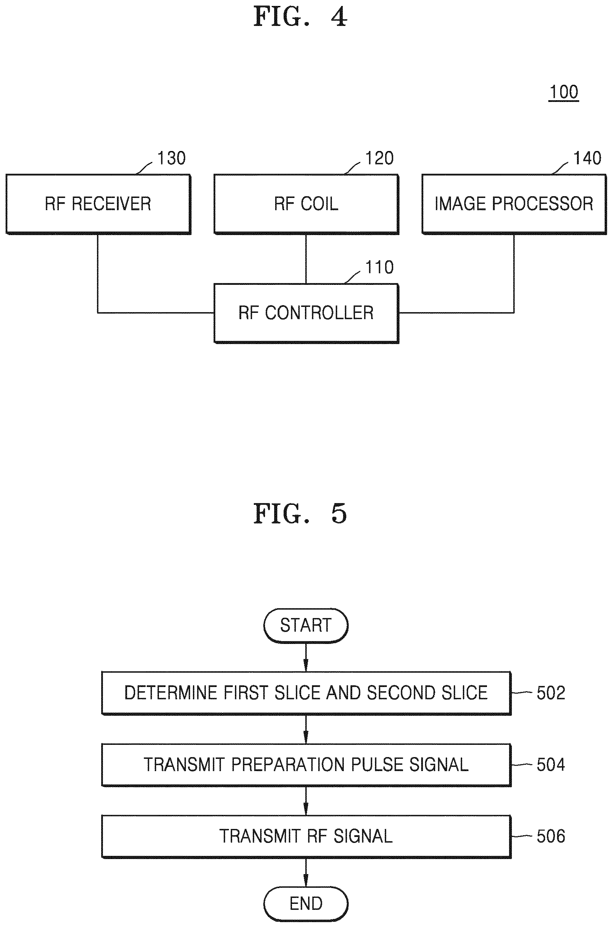

The preparation pulse signal may be a signal that is previously transmitted to adjust a contrast of an image to be acquired. For example, the preparation pulse signal may be an inverted pulse signal that is previously transmitted to acquire a fluid-attenuated inversion recovery (FLAIR) image. FIG. 4 is a diagram of the MRI apparatus 100 according to an embodiment. The MRI apparatus 100 may include the controller 110, the RF coil 120, and an image processor 140.

The controller 110 of FIG. 4 may correspond to the RF controller 56 of FIG. 1, the RF coil 120 of FIG. 4 may correspond to the RF coil 26 of FIG. 1, and the image processor 140 of FIG. 4 may correspond to the image processor 62 of FIG. 1.

FIG. 4 is a diagram illustrating an example of the MRI apparatus 100 of FIG. 3. Accordingly, although omitted, the description of the MRI apparatus 100 of FIG. 3 applies to the MRI apparatus 100 of FIG. 4.

In an embodiment, the RF coil 120 may receive MR signals of a first slice and a second slice. The first slice and the second slice of an object may emit the MR signals as an RF signal and a gradient magnetic field are applied. The gradient magnetic field may be applied by the gradient amplifier 32 of FIG. 1.

The RF coil 120 may receive the MR signals emitted by the first slice and the second slice. The RF coil 120 may acquire the MR signals emitted by the first slice and the second slice in an overlapping state during the same repetition time (TR) period.

The RF coil 120 may include a plurality of coils, and each of the plurality of coils may receive an MR signal. For example, the plurality of coils may include RF coils having various numbers of channels such as 16 channels, 32 channels, 72 channels, or 144 channels. The MR signals in the overlapping state may be distinguished based on a sensitivity difference between the plurality of coils.

The image processor 140 may acquire an MR image having a first contrast for the first slice, and an MR image having a second contrast for the second slice.

In an embodiment, the MR image having the first contrast may be a FLAIR image. Also, the MR image having the second contrast may be a T1-weighted image or a T2-weighted image.

In detail, the image processor 140 may receive the MR signals from the RF coil 120. The image processor 140 may generate 3D k-space data based on the received MR signals.

Also, the image processor 140 may acquire the MR images of the first slice and the second slice of the object based on the generated 3D k-space data. In detail, the image processor 140 may generate the 3D k-space data by performing 3D spatial encoding and performing 3D Fourier transform on the MR signals.

Also, the image processor 140 may acquire the MR images of the first slice and the second slice from the MR signals in the overlapping state by using at least one of parallel imaging and compressed sensing (CS).

For example, the image processor 140 may simultaneously acquire the MR signals from the first slice and the second slice through the plurality of coils by simultaneously exciting the first slice and the second slice according to the parallel imaging.

Also, the image processor 140 may separate the MR signals of the first slice and the second slice by using a difference in coil sensitivity information between the first slice and the second slice. The parallel imaging may include a SENSE method and a GRAPPA method. The CS may refer to a method of reconstructing an MR image after acquiring signals only for subsets instead of acquiring signals for all grids of a k-space.

When the 3D spatial encoding is performed on the MR signals, it may mean that digital data of the MR signals is arranged in a k-space of a memory. That is, the k-space may refer to a set of raw data of the MR signals and may include location information and contrast information.

Also, the k-space may refer to 2D or 3D Fourier transform of the measured MR signals. The image processor 140 may acquire the MR images by performing inverse 3D Fourier transform on the 3D k-space data.

The controller 110 may sequentially transmit RF signals to a plurality of slices of the object. Also, the controller 110 may transmit an RF signal including a frequency component that enables a slice corresponding to each of the plurality of slices to be excited.

For example, the controller 110 may control the RF coil 120 to sequentially transmit RF signals to odd-numbered slices of the object. In this case, the controller 110 may determine an even-numbered slice separated by a predetermined interval from each of the odd-numbered slices. The RF coil 120 may transmit an RF signal including a frequency component that enables each odd-numbered slice to be excited and a frequency component that enables the even-numbered slice separated by the predetermined interval from the odd-numbered slice to be excited.

The RF coil 120 may receive MR signals in an overlapping state from each odd-numbered slice and the even-numbered slice separated by the predetermined interval from the odd-numbered slice. The image processor 140 may receive the MR signals from the RF coil 120. The image processor 140 may acquire MR images in an overlapping state for each odd-numbered slice and the even-numbered slice separated by the predetermined interval from the odd-numbered slice, from the MR signals received from the RF coil 120.

The image processor 140 may acquire the MR image of each slice by separating the overlapping signals or the overlapping images.

In an embodiment, each MR image may include at least one from among a T1-weighted image, a T2-weighted image, a T2*-weighted image, a proton density (PD) image, and a FLAIR image.

FIG. 5 is a schematic flowchart of an MRI method according to an embodiment. The MRI method according to an embodiment may be performed by the MRI apparatus 100 of FIG. 3, and may perform the same operation as that of the MRI apparatus 100.

In operation 502, the MRI apparatus 100 determines a first slice and a second slice of an object. In an embodiment, the MRI apparatus 100 determines the first slice and the second slice so that a difference between a slice number of the first slice and a slice number of the second slice is a predetermined interval value.

In an embodiment, when the first slice is an even-numbered slice, the second slice may be an odd-numbered slice, and when the first slice is an odd-numbered slice, the second slice may be an even-numbered slice.

In an embodiment, the predetermined interval value may be a value obtained by dividing a total number of slices constituting the object by a multi-band acceleration factor. For example, when the multi-band acceleration factor is 2, the predetermined interval value may be half the total number of the slices constituting the object.

In another embodiment, the multi-band acceleration factor may be N. In this case, the MRI apparatus 100 may simultaneously acquire MR images for N (N>2) slices.

For example, the MRI apparatus 100 may transmit an RF signal including a frequency component that enables the N slices including the first slice and the second slice to be simultaneously excited. In this case, the predetermined interval value may be a value obtained by dividing the total number of the slices constituting the object by N.

In an embodiment, the MRI apparatus 100 may determine a plurality of slices including the first slice and the second slice of the object.

In operation 504, the MRI apparatus 100 transmits a preparation pulse signal including a frequency component that enables the first slice to be excited. The preparation pulse signal may be a signal that is previously transmitted to adjust a contrast of an image to be acquired. For example, the preparation pulse signal may be an inverted pulse signal that is previously transmitted to acquire a FLAIR image.

In operation 506, the MRI apparatus 100 transmits an RF signal including a plurality of frequency components so that both the first slice and the second slice are excited. In an embodiment, the MRI apparatus 100 may transmit the RF signal including the plurality of frequency components so that the plurality of slices including the first slice and the second slice are excited.

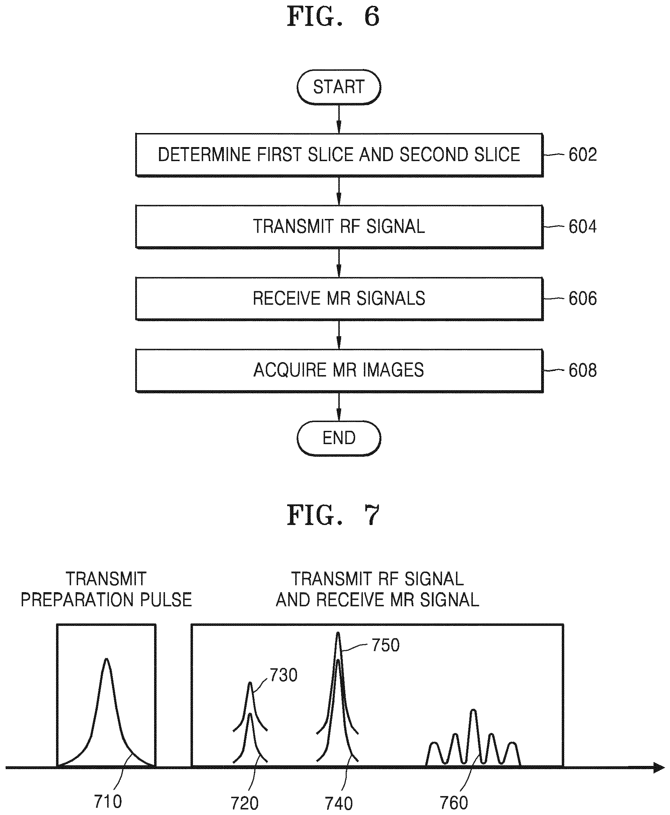

FIG. 6 is a flowchart of an MRI method according to an embodiment. In operation 602, the MRI apparatus 100 may determine a first slice and a second slice.

The MRI apparatus 100 may determine the first slice and the second slice so that a difference between a slice number of the first slice and a slice number of the second slice is a predetermined interval value. In an embodiment, the predetermined interval value may be a value obtained by dividing a total number of slices constituting an object by a multi-band acceleration factor. For example, when the multi-band acceleration factor is 2, the predetermined interval value may be half the total number of the slices constituting the object.

For example, the slice number of the first slice may be 1, and the total number of the slices may be 22. In this case, the predetermined interval value may be 11 that is half of 22. Accordingly, the MRI apparatus 100 may determine a slice whose slice number is 12 as the second slice.

In another embodiment, the multi-band acceleration factor may be N. In this case, the MRI apparatus 100 may simultaneously acquire MR images for N (N>2) slices.

For example, the MRI apparatus 100 may transmit an RF signal including a frequency component that enables the N slices including the first slice and the second slice to be simultaneously excited. In this case, the predetermined interval value may be a value obtained by dividing the total number of the slices constituting the object by N.

In operation 604, the MRI apparatus 100 may transmit a preparation pulse signal for the first slice. Also, the MRI apparatus 100 may transmit an RF signal including a plurality of frequency components so that both the first slice and the second slice are excited.

In an embodiment, the MRI apparatus 100 may transmit an inverted pulse signal for the first slice. In operation 606, the MRI apparatus 100 may receive MR signals of the first slice and the second slice. The MRI apparatus 100 may acquire the MR signals emitted from the first slice and the second slice in an overlapping state.

In operation 608, the MRI apparatus 100 may acquire MR images for the first slice and the second slice. In an embodiment, the MRI apparatus 100 may acquire a FLAIR image for the first slice. Also, the MRI apparatus 100 may acquire a T1-weighted image or a T2-weighted image for the second slice.

In an embodiment, the MRI apparatus 100 may acquire the MR image for the first slice and the MR image for the second slice in an overlapping state. The MRI apparatus 100 may acquire the MR image for each slice by separating the overlapping images.

FIG. 7 is a pulse sequence schematic diagram according to an embodiment.

The MRI apparatus 100 may transmit a preparation pulse signal 710. In an embodiment, the preparation pulse signal 710 may include a frequency component that enables a first slice to be excited.

In an embodiment, the preparation pulse signal 710 may be an inverted pulse signal used to acquire a FLAIR image.

The MRI apparatus 100 may transmit an RF signal including a plurality of frequency components so that both the first slice and a second slice are excited. Referring to FIG. 7, a method of reading an MR signal by using a spin echo method is illustrated. However, a method used to read an MR signal is not limited thereto.

In an embodiment, an RF signal may include a 90° pulse signal 720 including a frequency component that enables the first slice to be excited, and a 90° pulse signal 730 including a frequency component that enables the second slice to be excited.

Also, the RF signal may include a 180° pulse signal 740 including a frequency component that enables the first slice to be excited, and a 180° pulse signal 750 including a frequency component that enables the second slice to be excited.

The MRI apparatus 100 may receive MR signals 760 of the first slice and the second slice. The MRI apparatus 100 may acquire the MR signals 760 emitted from the first slice and the second slice in an overlapping state.

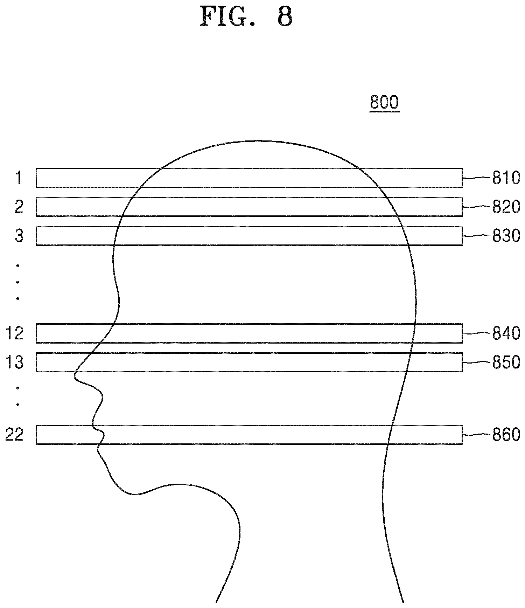

FIG. 8 is a diagram illustrating a plurality of slices of an object according to an embodiment. Referring to FIG. 8, the plurality of slices for acquiring an MR image of the head of the object are illustrated.

In an embodiment, the number of the plurality of slices of the object may be 22. Also, the slices may sequentially have slice numbers from 1 to 22. Referring to FIG. 8, the plurality of slices of the object include 22 slices from a slice 810 whose slice number is 1 to a slice 860 whose slice number is 22.

In the specification, a slice whose slice number is an odd number is an odd-numbered slice, and a slice whose slice number is an even number is an even-numbered slice.

In an embodiment, the MRI apparatus 100 may simultaneously acquire MR images having a plurality of contrasts for the plurality of slices.

In an embodiment, when a preparation pulse signal for the first slice is applied, slices around the first slice may be affected by the preparation pulse signal.

For example, when an inverted pulse signal for the slice 820 whose slice number is 2 is applied, phases of atomic nuclei included in the slice 820 whose slice number is 2 may be inverted. Also, due to the inverted pulse signal for the slice 820 whose slice number is 2, phases of atomic nuclei included in the slice 810 whose slice number is 1 and the slice 830 whose slice number is 3 may be changed.

Accordingly, when MR images having different contrasts for the first slice and a second slice are simultaneously acquired, the second slice that may be least effected by the preparation pulse signal for the first slice may be determined.

In an embodiment, a slice separated by half a total number of slices constituting the object from the first slice may be determined as the second slice. In detail, the second slice may be determined so that a difference between a slice number of the first slice and a slice number of the second slice is half the total number of the slices.

For example, when the first slice is the slice 810 whose slice number is 1, the second slice may be the slice 840 whose slice number is 12. Also, when the first slice is the slice 850 whose slice number is 13, the second slice may be the slice 820 whose slice number is 2.

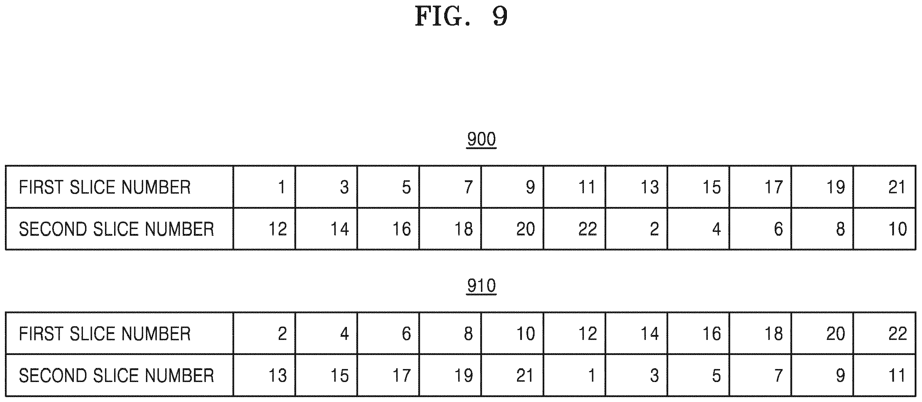

FIG. 9 illustrates tables each showing an order of selecting a first slice and a second slice according to an embodiment.

Table 1 900 is a table showing a case where a first slice is an odd-numbered slice. Table 2 910 is a table showing a case where a first slice is an even-numbered slice.

In an embodiment, the MRI apparatus 100 may acquire MR images having a plurality of contrasts for all slices constituting an object. For example, the MRI apparatus 100 may acquire a FLAIR image and a T2-weighted image.

In an embodiment, the MRI apparatus 100 may acquire a FLAIR image for an odd-numbered slice, and then may sequentially acquire a FLAIR image for an even-numbered slice. In another embodiment, the MRI apparatus 100 may acquire a FLAIR image for an even-numbered slice, and then may sequentially acquire a FLAIR image for an odd-numbered slice.

In an embodiment, when the MRI apparatus 100 acquires a FLAIR image for an odd-numbered slice, the MRI apparatus 100 may simultaneously acquire a T2-weighted image for an even-numbered slice.

In an embodiment, the MRI apparatus 100 may acquire a FLAIR image for a first slice that is an odd-numbered slice. In this case, the MRI apparatus 100 may simultaneously acquire a T2-weighted image for a second slice that is an even-numbered slice that may be least affected by an inverted pulse signal.

In an embodiment, the MRI apparatus 100 may determine a second slice by using Equation 1.

$$slice\ number\ of\ second\ slice = \frac{slice\ number\ of\ first\ slice + total\ number\ of\ slices}{multiband\ factor} \% \ total\ number\ of\ slices \quad \dots (1)$$

In Equation 1, a multi-band factor may be the number of slices to be simultaneously excited by using an RF signal. In detail, the multi-band factor may refer to the number of frequencies included in the RF signal.

For example, when a FLAIR image for a first slice and a T2 image for a second slice are to be simultaneously acquired, the multi-band factor is 2.

In another embodiment, the MRI apparatus 100 may simultaneously acquire MR images for N (N>2) slices. In this case, the MRI apparatus 100 may transmit an RF signal including a frequency component that enables the N slices including a first slice and a second slice to be simultaneously excited. In this case, the multi-band factor is N.

Referring to FIGS. 8 and 9, the MRI apparatus 100 may determine a second slice by using Equation 2.

$$slice\ number\ of\ second\ slice = (slice\ number\ of\ first\ slice + 11)\%22 \dots (2)$$

For example, when a slice number of a first slice is 1, a slice number of a second slice may be 12. Also, when a slice number of the first slice is 17, a slice number of the second slice may be 6. Referring to FIG. 9, a result obtained after selecting the first slice and the second slice corresponding to the first slice according to an embodiment is illustrated.

The embodiments may be written as computer programs and may be implemented in general-use digital computers that execute the programs using a computer-readable recording medium. Examples of the computer-readable recording medium include magnetic storage media (e.g., read-on memories (ROMs), floppy disks, hard disks, etc.), optical recording media (e.g., compact disk (CD)-ROMs or digital versatile disks (DVDs)), and carrier waves (such as data transmission through the Internet).

While the present disclosure has been particularly shown and described with reference to embodiments thereof, it will be understood by one of ordinary skill in the art that various changes in form and details may be made therein without departing from the spirit and scope of the present disclosure as defined by the following claims.

**EP 3 409 192 A1**

Sequence Listing Free Text

100: MRI apparatus
110: controller
120: RF coil
140: image processor

**Claims**

1. A magnetic resonance imaging (MRI) apparatus comprising:

   a controller configured to determine a first slice and a second slice so that a difference between a slice number of the first slice of an object and a slice number of the second slice of the object is a predetermined interval value; and
   a radio frequency (RF) coil configured to transmit a preparation pulse signal comprising a frequency component enabling the first slice to be excited and transmit an RF signal comprising a plurality of frequency components so that both the first slice and the second slice are excited.

2. The MRI apparatus of claim 1, wherein the controller is further configured to determine the first slice and the second slice so that when the first slice is an even-numbered slice, the second slice is an odd-numbered slice, and when the first slice is an odd-numbered slice, the second slice is an even-numbered slice.

3. The MRI apparatus of claim 1, wherein the controller is further configured to determine the first slice and the second slice so that the difference between the slice number of the first slice and the slice number of the second slice is half a total number of slices constituting the object.

4. The MRI apparatus of claim 1, wherein the RF coil is further configured to receive magnetic resonance (MR) signals of the first slice and the second slice,
   wherein the MRI apparatus further comprises an image processor configured to acquire an MR image having a first contrast for the first slice and an MR image having a second contrast for the second slice.

5. The MRI apparatus of claim 4, wherein the RF coil is further configured to transmit an inverted RF pulse signal comprising a frequency component enabling the first slice to be excited.

6. The MRI apparatus of claim 5, wherein the image processor is further configured to acquire a fluid-attenuated inversion recovery (FLAIR) image for the first slice and acquire at least one image from among a T1-weighted image and a T2-weighted image for the second slice.

7. The MRI apparatus of claim 4, wherein the image processor is further configured to sequentially acquire images having the first contrast for a plurality of slices comprising the first slice, and simultaneously acquire images having the second contrast for slices respectively corresponding to the plurality of slices in a state where the images having the second contrast overlap the images having the first contrast,
   wherein a difference between a slice number of each of the corresponding slices and a slice number of each of the plurality of slices is the predetermined interval value.

8. The MRI apparatus of claim 7, wherein each of the images having the first contrast is a FLAIR image, and each of the images having the second contrast is a T1-weighted image or a T2-weighted image.

9. The MRI apparatus of claim 7, wherein the predetermined interval value is a value obtained by dividing a total number of slices constituting the object by a multi-band acceleration factor.

10. A magnetic resonance imaging (MRI) method comprising:

    determining a first slice and a second slice of an object;
    transmitting a preparation pulse signal comprising a frequency component enabling the first slice to be excited;

12

and
transmitting a radio frequency (RF) signal comprising a plurality of frequency components so that both the first slice and the second slice are excited,
wherein the determining comprises determining the first slice and the second slice so that a difference between a slice number of the first slice and a slice number of the second slice is a predetermined interval value.

11. The MRI method of claim 10, wherein the determining comprises determining the first slice and the second slice so that when the first slice is an even-numbered slice, the second slice is an odd-numbered slice, and when the first slice is an odd-numbered slice, the second slice is an even-numbered slice.

12. The MRI method of claim 10, wherein the determining comprises determining the first slice and the second slice so that the difference between the slice number of the first slice and the slice number of the second slice is half a total number of slices constituting the object.

13. The MRI method of claim 10, further comprising:

receiving magnetic resonance (MR) signals of the first slice and the second slice; and
acquiring an MR image having a first contrast for the first slice and an MR image having a second contrast for the second slice.

14. The MRI method of claim 13, wherein the transmitting of the preparation pulse signal comprises transmitting an inverted RF pulse signal comprising a frequency component enabling the first slice to be excited.

15. The MRI method of claim 14, wherein the acquiring comprises:

acquiring a fluid-attenuated inversion recovery (FLAIR) image for the first slice; and
acquiring at least one image from among a T1-weighted image and a T2-weighted image for the second slice.

16. The MRI method of claim 13, further comprising sequentially acquiring images having the first contrast for a plurality of slices comprising the first slice,
wherein the sequentially acquiring comprises simultaneously acquiring images having the second contrast for slices respectively corresponding to the plurality of slices in a state where the images having the second contrast overlap the images having the first contrast,
wherein a difference between a slice number of each of the corresponding slices and a slice number of each of the plurality of slices is the predetermined interval value.

17. The MRI method of claim 16, wherein each of the images having the first contrast is a FLAIR image, and each of the images having the second contrast is a T1-weighted image or a T2-weighted image.

18. The MRI method of claim 16, wherein the predetermined interval value is a value obtained by dividing a total number of slices constituting the object by a multi-band acceleration factor.

19. A computer-readable recording medium having embodied thereon a program for executing the MRI method of claim 10.

FIG. 1

EP 3 409 192 A1

# FIG. 2

COMMUNICATION UNIT /70

LOCAL AREA COMMUNICATION MODULE /72

WIRED COMMUNICATION MODULE /74

WIRELESS COMMUNICATION MODULE /76

NETWORK /80

SERVER —92

MEDICAL APPARATUS —94

PORTABLE DEVICE —96

# FIG. 3

100

RF COIL /120

RF CONTROLLER /110

# FIG. 4

<u>100</u>

| 130 RF RECEIVER | 120 RF COIL | 140 IMAGE PROCESSOR |
|---|---|---|

110 RF CONTROLLER

# FIG. 5

START

DETERMINE FIRST SLICE AND SECOND SLICE — 502

TRANSMIT PREPARATION PULSE SIGNAL — 504

TRANSMIT RF SIGNAL — 506

END

# FIG. 6

START

DETERMINE FIRST SLICE AND SECOND SLICE — 602

TRANSMIT RF SIGNAL — 604

RECEIVE MR SIGNALS — 606

ACQUIRE MR IMAGES — 608

END

# FIG. 7

TRANSMIT
PREPARATION PULSE

TRANSMIT RF SIGNAL
AND RECEIVE MR SIGNAL

710

720    730    740    750    760

FIG. 8

# FIG. 9

EP 3 409 192 A1

900

| FIRST SLICE NUMBER | 1 | 3 | 5 | 7 | 9 | 11 | 13 | 15 | 17 | 19 | 21 |
|---|---|---|---|---|---|---|---|---|---|---|---|
| SECOND SLICE NUMBER | 12 | 14 | 16 | 18 | 20 | 22 | 2 | 4 | 6 | 8 | 10 |

910

| FIRST SLICE NUMBER | 2 | 4 | 6 | 8 | 10 | 12 | 14 | 16 | 18 | 20 | 22 |
|---|---|---|---|---|---|---|---|---|---|---|---|
| SECOND SLICE NUMBER | 13 | 15 | 17 | 19 | 21 | 1 | 3 | 5 | 7 | 9 | 11 |

## INTERNATIONAL SEARCH REPORT

| | International application No. |
|---|---|
| | **PCT/KR2016/010811** |

| A. | CLASSIFICATION OF SUBJECT MATTER |
|---|---|

*A61B 5/055(2006.01)i, A61B 5/00(2006.01)i, G01R 33/36(2006.01)i, G01R 33/54(2006.01)i, G01R 33/56(2006.01)i*

According to International Patent Classification (IPC) or to both national classification and IPC

| B. | FIELDS SEARCHED |
|---|---|

Minimum documentation searched (classification system followed by classification symbols)
A61B 5/055; G01R 33/561; G01R 33/48; A61B 5/00; G01R 33/36; G01R 33/54

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: contrast, slice, MRI, preparation, pulse

| C. | DOCUMENTS CONSIDERED TO BE RELEVANT | |
|---|---|---|
| Category* | Citation of document, with indication, where appropriate, of the relevant passages | Relevant to claim No. |
| Y | US 2014-0225612 A1 (POLIMENI et al.) 14 August 2014<br>See abstract, paragraph [60], claims 1-13 and figure 4B. | 1-3,19 |
| A | | 4-9 |
| Y | KR 10-0732790 B1 (KABUSHIKI KAISHA TOSHIBA) 27 June 2007<br>See abstract, claims 1-5 and figures 5-7. | 1-3,19 |
| A | US 2012-0046539 A1 (VISSER) 23 February 2012<br>See abstract, paragraphs [32], [33] and figure 2. | 1-9,19 |
| A | US 2011-0254548 A1 (SETSOMPOP et al.) 20 October 2011<br>See abstract and claims 1-4. | 1-9,19 |
| A | US 2014-0253120 A1 (REGENTS OF THE UNIVERSITY OF MINNESOTA)<br>11 September 2014<br>See abstract, claims 1-13 and figures 3A-3E. | 1-9,19 |

| ☐ Further documents are listed in the continuation of Box C. | ☒ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 JANUARY 2017 (06.01.2017) | **06 JANUARY 2017 (06.01.2017)** |

| Name and mailing address of the ISA/KR<br>Korean Intellectual Property Office<br>Government Complex-Daejeon, 189 Seonsa-ro, Daejeon 302-701,<br>Republic of Korea<br>Facsimile No. +82-42-481-8578 | Authorized officer<br><br>Telephone No. |
|---|---|

Form PCT/ISA/210 (second sheet) (January 2015)

## INTERNATIONAL SEARCH REPORT

| International application No. |
|---|
| PCT/KR2016/010811 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: **10-18**
   because they relate to subject matter not required to be searched by this Authority, namely:

   Claims 10 to 18 pertain to a method for diagnosis, and thus pertain to subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.

**PCT/KR2016/010811**

| Patent document cited in search report | Publication date | Patent family member | Publication date |
|---|---|---|---|
| US 2014-0225612 A1 | 14/08/2014 | NONE | |
| KR 10-0732790 B1 | 27/06/2007 | EP 1444949 A1 | 11/08/2004 |
| | | EP 1444949 B1 | 24/01/2007 |
| | | KR 10-0646914 B1 | 17/11/2006 |
| | | KR 10-2006-0090312 A | 10/08/2006 |
| | | US 2004-0049106 A1 | 11/03/2004 |
| | | US 7623901 B2 | 24/11/2009 |
| | | WO 03-041579 A1 | 22/05/2003 |
| US 2012-0046539 A1 | 23/02/2012 | CN 102378910 A | 14/03/2012 |
| | | EP 2414819 A1 | 08/02/2012 |
| | | JP 2012-522560 A | 27/09/2012 |
| | | WO 2010-113083 A1 | 07/10/2010 |
| US 2011-0254548 A1 | 20/10/2011 | US 2013-0181710 A1 | 18/07/2013 |
| | | US 8405395 B2 | 26/03/2013 |
| US 2014-0253120 A1 | 11/09/2014 | WO 2013-052535 A1 | 11/04/2013 |

Form PCT/ISA/210 (patent family annex) (January 2015)